Europäisches Patentamt

(19) European Patent Office

Office européen des brevets

(11) Veröffentlichungsnummer: **0 002 747**
**B1**

(12) **EUROPÄISCHE PATENTSCHRIFT**

(45) Veröffentlichungstag der Patentschrift:
07.01.81

(51) Int. Cl.³: **C 07 D 235/26, C 09 B 29/20**

(21) Anmeldenummer: **78101709.0**

(22) Anmeldetag: **15.12.78**

(54) Neue Kristallmodifikation des 5-(2'-Hydroxy-3'-naphthoylamino)-benzimidazolon-(2), Verfahren zu ihrer Herstellung und ihre Verwendung.

(30) Priorität: 30.12.77 DE 2758818

(43) Veröffentlichungstag der Anmeldung:
11.07.79 Patentblatt 79/14

(45) Bekanntmachung des Hinweises auf die Patenterteilung:
07.01.81 Patentblatt 81/1

(84) Benannte Vertragsstaaten:
BE CH DE FR GB IT

(56) Entgegenhaltungen:
DE-A-2 110 349
DE-A-2 441 524
DE-C-1 188 229
DE-C-2 405 986
W. FOERST: "Ullmanns Encyklopädie der technischen Chemie", 5. Auflage, 12. Band, 1960,
Urban & Schwarzenberg
München – Berlin
Seiten 607 bis 609

(73) Patentinhaber: HOECHST AKTIENGESELLSCHAFT,
Zentrale Patentabteilung Postfach 80 03 20,
D-6230 Frankfurt/Main 80 (DE)

(72) Erfinder: Arndt, Otto, Dr., Frankfurter Strasse 38,
D-6238 Hofheim am Taunus (DE)
Erfinder: Mees, Bernhard, Dr., Gräfliche Strasse 31,
D-6239 Eppstein/Taunus (DE)
Erfinder: Tronich, Wolfgang, Dr.,
Martin-Wohmann-Strasse 27, D-6238 Hofheim am Taunus (DE)
Erfinder: Dietz, Erich, Dr., Brunhildenweg 2,
D-6233 Kelkheim (Taunus) (DE)

# Neue Kristallmodifikation des 5-(2'-Hydroxy-3'naphthoyl-amino)-benzimidazolon-(2), Verfahren zu ihrer Herstellung und ihre Verwendung

Die Verwendung von 5-(2'-Hydroxy-3'-naphthoyl-amino-benzimidazolon-(2) als Kupplungskomponente für Azopigmente ist beispielsweise aus der DE-PS 1 188 229 bekannt. In dieser PS finden sich jedoch keine Angaben für die Herstellung der Kupplungskomponente. Stellt man diese Verbindung durch Kondensation von 2-Hydroxy-3-naphthoesäure mit 5-Aminobenzimidazolon-(2) nach üblichen Methoden her, wie sie beispielsweise in der DE-PS 2405986 beschrieben sind, nämlich in einem unpolaren organischen Lösemittel, so erhält man ein ziemlich unreines, schlecht filtrierbares Produkt. Dieses Rohprodukt erfüllt nicht die Anforderungen, die an ein Ausgangsmaterial zur Herstellung hochechter Pigmente gestellt werden und muss deshalb in einem weiteren Reinigungsverfahren aufbereitet werden. Hierzu wird das Rohmaterial im allgemeinen mittels wässriger Alkalilauge in das Alkalisalz überführt und aus dessen Lösung mit einer Mineralsäure ausgefällt. Das so erhaltene Fällungsprodukt wird in Form grauer bis weisser Kristalle, im folgenden «weisse Modifikation» genannt, erhalten und eignet sich zur Herstellung von Pigmenten.

Abgesehen von der aufwendigen Reinigung des Rohprodukts, die mit einem zusätzlichen Zeitaufwand und Ausbeuteverlusten verbunden ist und eine Abwasseraufarbeitung erforderlich macht, ist das bekannte Verfahren auch noch insofern nachteilig, als man erhebliche Mengen an Lösemittel braucht, um eine rührfähige Suspension während der Kondensation zu gewährleisten. Darüber hinaus benötigt das bekannte Verfahren sehr lange Reaktionszeiten und ergibt unbefriedigende Ausbeuten. Ausserdem ist das Produkt schlecht filtrierbar.

Es wurde nun gefunden, dass 5-(2'-Hydroxy-3'-naphthoyl-amino)-benzimidazolon-(2) noch in einer weiteren Kristallmodifikation existiert, die im folgenden aufgrund ihrer Farbe als «gelbe Modifikation» bezeichnet werden soll. Diese neue Modifikation ist gekennzeichnet durch ein Kristallgitter mit folgenden Netzebenenabständen (in $10^{-10}$ m):

3,12; 3,26; 3,37; 3,46; 3,58; 3,75; 4,64; 5,26; 5,43; 5,93; 6,47; 8,74 und 13,10.

Diese neue gelbe Modifikation zeichnet sich gegenüber der bekannten, «weissen» Modifikation dadurch aus, dass sie leicht in sehr reiner Form zugänglich ist, die direkt zur Herstellung hochwertiger Azopigmente eingesetzt werden kann. Darüberhinaus zeichnet sich die gelbe Modifikation dadurch aus, dass sie in derben, schuppenförmigen Kristallgebilden anfällt, die nicht die Neigung zur Agglomerisation zeigen und daher in Suspensionen leicht rührbar und ausgesprochen leicht filtrierbar sind.

Die erfindungsgemässe gelbe Kristallmodifikation wird in einfacher Weise dadurch erhalten, dass man 2-Hydroxy-3-naphtheosäure mit 5-Amino-benzimidazolon-(2) in einem organischen, polar aprotischen Lösemittel bei Temperaturen über 100°C kondensiert. Das aus diesem Reaktionsmedium kristallisierende Produkt schliesst weder Verunreinigungen aus der Reaktionsmischung ein noch werden solche Verunreinigungen adsorbiert. Darüberhinaus zeigt das Verfahren eine vorzügliche Raum-Zeit-Ausbeute und es fällt kein Abwasser an. Das Lösemittel kann aus der Mutterlauge nach bekannten Verfahren einfach zurückgewonnen werden.

Bevorzugt wählt man als Reaktionsmedium ein Lösemittel, in dem die Ausgangsmaterialien unter den Reaktionsbedingungen vollständig gelöst sind, aus dem aber andererseits das Produkt leicht kristallisiert und aus dessen Mutterlauge das Lösemittel gut wieder zu gewinnen ist. Bevorzugte Lösemittel sind Dimethylformamid, Dimethylacetamid, Dimethylsulfoxid, Tetramethylensulfon (Sulfolan), Tetramethylharnstoff, Hexamethylphosphorsäuretrisamid und insbesondere N-Methylpyrrolidon, aber auch beispielsweise Picoline. Im Vergleich zu den nach dem Stand der Technik verwendeten unpolaren Lösemitteln sind von den erfindungsgemäss eingesetzten polar aprotischen Lösemitteln nur relativ geringe Mengen erforderlich, was zur Verbesserung der Raum-Zeit-Ausbeute beiträgt.

Im folgenden werden einige bevorzugte Ausgestaltungen des erfindungsgemässen Verfahrens näher beschrieben:

Die 2-Hydroxy-3-naphthoesäure wird in dem genannten Lösemittel mit einer anorganischen oder organischen Base teilweise oder vollständig in ein Salz übergeführt. Als anorganische Basen können die Hydroxide oder Carbonate der Alkalimetalle eingesetzt werden, vorzugsweise Natriumhydroxid oder Natriumcarbonat. Als organische Basen kommen Stickstoffheterocyclen, wie beispielsweise Pyridin oder Picoline in Frage, die gegebenenfalls auch als Lösemittel dienen können. Auch bei Verwendung von N-Methylpyrrolidon als Kondensationsmedium ist der Zusatz einer Base nicht erforderlich, führt aber zu höheren Ausbeuten. Das bei der Verwendung eines Hydroxids entstehende Neutralisationswasser wird aus dem Reaktionsmedium abgetrennt, vorzugsweise im Vakuum abdestilliert.

Das eingesetzte 5-Aminobenzimidazolon-(2) kann als trockener Feststoff, als wasserhaltiger Filterkuchen oder als Lösung in dem polar aprotischen, organischen Lösemittel vor oder nach der Neutralisation der 2-Hydroxy-3-naphthoesäure zugesetzt werden.

Wenn eine Abtrennung von Wasser erforderlich ist, so wird diese zweckmässig als destillative Entwässerung im Vakuum von 5 bis 150 mbar Stickstoff, vorzugsweise jedoch bei 20 bis 80 mbar, im Temperaturbereich von 60 bis 140°C, insbesondere 85 bis 120°C durchgeführt.

Die 2-Hydroxy-3-naphthoesäure wird im allgemeinen im Überschuss von bis zu 20 Mol-% eingesetzt; wenn eine Base zugesetzt wird, so werden hiervon bis zu etwa 120 Äquivalent-%, bezogen auf 5-Aminobenzimidazolon-(2), vorzugsweise jedoch 80–120 Äquivalent-% eingesetzt.

Das polar aprotische, organische Lösemittel wird in einer solchen Menge eingesetzt, dass nach einer eventuellen Entwässerung 200 bis 400, bevorzugt 300 Gew.-%, bezogen auf das Produkt, vorliegen.

Die Umsetzung wird vorzugsweise in einem Einstufen- bzw. Eintopfverfahren durchgeführt, wobei ein übliches saures Kondensationsmittel zugesetzt wird. Sofern eine Entwässerung erfolgt, wird das Kondensationsmittel unmittelbar nach der Entwässerung zugegeben. Als solche üblichen sauren Kondensationsmittel verwendet man vorzugsweise die Halogenide starker Mineralsäuren, insbesondere Phosphortrichlorid, in einer Einsatzmenge bis zu 60 Mol-%, bezogen auf das 5-Amino-benzimidazolon-(2), insbesondere 30 bis 60%, vor allem 47 Mol-% Phosphortrichlorid.

Es ist auch möglich, wenn auch im allgemeinen nicht besonders vorteilhaft, die Umsetzung in einem Zweistufen-Verfahren durchzuführen. Hierbei kann man zunächst das 5-Aminobenzimidazolon-(2) mit Phosphortrichlorid umsetzen und die so erhaltene Phosphorverbindung mit 2-Hydroxy-3-naphthoesäure reagieren lassen. Eine andere Ausführungsform des Zweistufen-Verfahrens besteht darin, dass man aus der 2-Hydroxy-3-naphthoesäure zunächst das Säurechlorid herstellt und dieses dann mit dem 5-Amino-benzimidazolon-(2) umsetzt.

Bei einem besonders bevorzugten Eintopf-Verfahren gibt man zu dem entwässerten Gemisch aus dem Natriumsalz der 2-Hydroxy-3-naphthoesäure und dem 5-Amino-benzimidazolon-(2) das Kondensationsmittel, insbesondere Phosphortrichlorid, bei Temperaturen von 20 bis 150°C, und führt die Reaktion entweder unter isothermen Bedingungen zunächst bei vorzugsweise 105 bis 110°C, dann bei 125 bis 150°C, vor allem 140 bis 150°C, oder unter adiabatischen Bedingungen bei vorzugsweise 105 bis 150°C, vor allem bei 140 bis 150°C durch.

Das Produkt kristallisiert im Verlauf der Kondensation, zweckmässig bei Temperaturen von 105 bis 120°C, vorzugsweise bei 110 bis 115°C aus. Die so erhaltene dünne bis mässig dicke, gut rührbare, zitronengelbe Suspension wird 1 bis 4, vorzugsweise 2 Stunden bei 120 bis 150°C, vorzugsweise 145°C nachgerührt. Anschliessend wird sie zur Vervollständigung der Kristallisation des Produkts langsam mit Wasser bei 110 bis 70°C versetzt.

Das abgetrennte Produkt wird dann bei Temperaturen von 20 bis 90°C, vorzugsweise 50°C filtriert und nacheinander mit einem Gemisch aus dem organischen Lösemittel und Wasser, dann Wasser, einer wässrigen Alkalilösung, vorzugsweise einer Lösung eines Alkalihydrogencarbonats, und abschliessend nochmals Wasser gewaschen. Die Ausbeuten liegen in der Grössenordnung von 95% der Theorie.

Es wurde weiterhin gefunden, dass auch die eingangs erwähnte «weisse» Modifikation, die nach Kondensation in einem unpolaren Lösemittel und Umfällung aus wässrig-alkalischer Lösung mit Säuren erhalten wird, in die erfindungsgemässe «gelbe» Modifikation übergeführt werden kann, wenn sie auf Temperaturen von 100 bis 300°C etwa 1 bis 8 Stunden lang erhitzt wird. Vorzugsweise wird hierbei nicht die trockene Substanz, sondern eine Suspension in geeigneten organischen Flüssigkeiten erhitzt. Vorteilhaft wählt man hierbei Flüssigkeiten, die eventuelle Verunreinigungen in der weissen Form lösen, insbesondere organische, polare aprotische Lösemittel, beispielsweise N-Methylpyrrolidon, Dimethylformamid, Picolin oder Gemische solcher Lösemittel.

Im folgenden sind bevorzugte Ausgestaltungen dieses erfindungsgemässen Verfahrens näher erläutert:

Die «weisse» Modifikation wird als trockene Substanz oder als wässriger Filterkuchen in eine Suspension in einem geeigneten Lösemittel, vorzugsweise N-Methylpyrrolidon, überführt und etwa 2 Stunden auf 100 bis 150°C, vorzugsweise 130°C erhitzt. Hierbei wird nur so viel Lösemittel eingesetzt, dass in der Hitze der grösste Teil des Feststoffs ungelöst ist, die Suspension aber noch gut rührbar ist. Im Falle des bevorzugten N-Methylpyrrolidons werden bis zu 10 Gewichtsteile pro Gewichtsteil Ausgangsmaterial eingesetzt. Selbstverständlich hängen die zur quantitativen Umwandlung erforderlichen Zeiten von der Temperatur ab und liegen zwischen wenigen Minuten bei etwa 150°C und mehreren Stunden bei 100°C. Wenn das Ausgangsmaterial in Form eines wässrigen Filterkuchens eingesetzt wird, wird das Wasser vorher nach üblichen Methoden abgetrennt. Das Ende der Umwandlung kann durch Entnahme von Proben und deren mikroskopische und röntgenografische Beurteilung festgestellt werden.

In den folgenden Beispielen beziehen sich Teile und Prozentangaben auf das Gewicht, sofern nichts anderes angegeben ist.

Beispiel 1

In 550 Teilen N-Methylpyrrolidon (=NMP) löst man 75 Teile 5-Aminobenzimidazolon-(2) (als ca. 70%ige wasserfeuchte Ware) und 115 Teile 2-Hydroxy-3-naphthoesäure. Man neutralisiert teilweise mit 23 Teilen Natriumcarbonat und destilliert im Vakuum von 20 bis 80 mbar Stickstoff bei bis zu 120°C Sumpftemperatur das Wasser (in Form von ca. 150 Teilen Wasser/NMP-Gemisch) ab. Der Gehalt an NMP im abdestillierten $H_2O$/NMP-Gemisch wird anhand des Brechungsindex mittels einer Eichkurve ermittelt und das abdestillierte NMP in Form von reinem NMP ergänzt (im allgemeinen ca. 50 Teile).

Anschliessend tropft man innerhalb von 20–30 Minuten 32 Teile reines, HCl-freies Phosphortrichlorid unter schwacher Kühlung auf die Oberfläche des Reaktionsgemisches so zu, dass die Temperatur zwischen 105–110°C bleibt. Nach weiteren 10–30 Minuten wird auf 145°C geheizt. Man rührt 2 Stunden bei dieser Temperatur nach, kühlt auf ca. 100–110°C ab und lässt langsam 500 Teile Wasser zulaufen. Man filtriert das Produkt bei 50°C ab und wäscht es bei 50°C nacheinander mit 300 Teilen NMP-Wasser-Gemisch (1:1), 500 Teilen Wasser, 500 Teilen einer 2%igen Lösung von Natriumhydrogencarbonat und nochmals 500 Teilen Wasser. Man erhält 150 Teile 5-(2′-Hydroxy-3′-naphthoyl-amino)-benzimidazolon-(2), im folgenden «Naphtholon» genannt, (=94% d.Th.) als ca. 60–70%iges wasserhaltiges Produkt mit gelbem Aspekt. Das Produkt wird im Vakuum bei 60–120°C getrocknet.

Das Röntgenbeugungsspektrum (CuK$_\alpha$) weist Reflexe bei den folgenden Glanzwinkeln auf (Intensitäten charakterisiert durch w = schwach, m = mittel, s = stark, vs = sehr stark):

3,4(m), 5,1(m), 6,85(vs), 7,5(m), 8,2(s), 8,45(w), 9,6(w), 11,9(w), 12,45(m), 12,9(s), 13,2(s), 13,7(m) und 14,3(w).

Hieraus ergeben sich folgende Netzebenenabstände ($10^{-10}$ m):

13,1; 8,74; 6,47; 5,93; 5,43; 5,26; 4,64; 3,75; 3,58; 3,46; 3,37; 3,26 und 3,12.

## Beispiel 2

Beispiel 1 wird wiederholt, jedoch werden statt Natriumcarbonat 68 Teile 33%ige Natronlauge zur teilweisen Neutralisation der 2-Hydroxy-3-naphthoesäure eingesetzt. Ausbeute und Röntgenbeugungsspektrum entsprechen den in Beispiel 1 gemachten Angaben.

## Beispiel 3

In 1300 Teilen Tetramethylensulfon (Sulfolan) werden 75 Teile 5-Aminobenzimidazolon-(2) (als ca. 70%ige wasserfeuchte Ware), 115 Teile 2-Hydroxy-3-naphthoesäure und 68 Teile 33%ige Natronlauge eingetragen. Nach Abdestillation des Wassers bis zu einer Sumpftemperatur von 135°C bei 15 mbar laufen 32 Teile Phosphortrichlorid zu. Nach kurzer Zeit kristallisiert das Naphtoholon in seiner gelben Modifikation. Man rührt die Suspension noch 3 Stunden bei 135°C nach und und filtriert bei 50°C durch eine Drucknutsche. Man wäscht mit 300 Teilen Sulfolan, dann mit 1000 Teilen Wasser und trocknet das Nutschgut bei 120°C im Vakuum. Man erhält 150 Teile trockenes Naphtholon (entspr. 94% d. Th.). Das Röntgenspektrum entspricht den unter Beispiel 1 gemachten Angaben.

## Beispiel 4

In einem emaillierten Rührkessel (360 l) werden 110 kg NMP (techn.) vorgelegt und nacheinander 16 kg 5-Aminobenzimidazolon-(2) (technisch feucht, Gehalt 93%), 23 kg 2-Hydroxy-3-naphthoesäure und 4,5 kg wasserfreies Natriumcarbonat eingetragen.

Nun wird bis zu einer Innentemperatur von 105°C bei einem Endvakuum von 15 mbar ein Gemisch von Wasser und NMP abdestilliert, bis der Brechungsindex des ablaufenden Destillats am Schluss 1,4700 bei 25°C beträgt. Man ergänzt das mit dem Wasser abdestillierte NMP im Ansatz auf 90 kg nach Massgabe der Destillatanalyse (Brechungsindex). Dann lässt man innerhalb von etwa 20 min 6,4 kg Phosphortrichlorid auf die Oberfläche des Reaktionsgemischs laufen, wobei die Temperatur auf 135–145°C ansteigt und das Produkt auszukristallisieren beginnt. Man rührt 2 Stunden bei 145°C nach, verdünnt die gelbe Suspension bei 80–100°C mit 150 l Trinkwasser und drückt sie aus dem Kessel in eine Drucknutsche (Steinfilter). Nach dem Filtrieren wäscht man den Nutschkuchen mit 60 kg NMP/Wasser-Gemisch (1:1), dann 3 mal mit je 100 l Trinkwasser, wobei dem 2. Waschwasser 2 kg Natriumhydrogencarbonat beigegeben sind. Der Filterkuchen wird trocken geblasen und bei 60–80°C im Vakuum getrocknet. Man erhält ca. 29 kg Naphtholon (techn., trocken 91% d. Th.). Aus der Mutterlauge und der Waschlauge wird nach Neutralisation das Lösemittel regeneriert.

## Beispiel 5

149 Teile 5-Aminobenzimidazolon-(2) (als ca. 70%ige wasserfeuchte Ware) werden in 1500 Teilen NMP gelöst. Die Lösung wird durch Andestillation im Vakuum von 20 mbar über eine Silbermantel-Vigreuxkolonne (40 cm) bei einem Rücklaufverhältnis 3:1 bis zur konstanten Kopftemperatur entwässert. Zu dem mit Stickstoff begasten Ansatz laufen 400 Teile Picolin (Gemisch aus α-, β- und γ-Picolin) und anschliessend 75 Teile Phosphortrichlorid bei 60°C. Man heizt innerhalb 1 Stunde auf 130°C und trägt dann 207 Teile 2-Hydroxy-3-naphthoesäure ein.

Man rührt 3 Stunden bei 130–140°C nach, fällt das Naphtholon mit 1000 Teilen Wasser in 1 Stunde aus und isoliert es durch Filtration. Das Waschen und Trocknen werden wie bei Beispiel 1 ausgeführt. Man erhält 260 Teile gelbes Naphtholon mit einem Reingehalt von ca. 98% in einer Ausbeute von 82° d. Th., bezogen auf 5-Aminobenzimidazolon-(2).

## Beispiel 6

149 Teile wasserfreies 5-Aminobenzimidazolon-(2) werden in 2000 Teilen Picolin (Gemisch wie in Beispiel 5) suspendiert und bei 65°C mit 75 Teilen Phosphortrichlorid versetzt. Man erhitzt zwei Stunden zum Rückfluss (127°C). Dabei bilden sich zwei flüssige Phasen. Anschliessend werden 207 Teile 2-Hydroxy-3-naphthoesäure eingetragen und weitere 3 Stunden unter Rückfluss erhitzt. Dabei bildet sich eine Suspension von gelbem Naphtholon. Zu der gelben Suspension laufen innerhalb 1 Stunde bei 110°C 500 Teile Wasser. Das Naphtholon wird filtriert, nacheinander mit Picolin/Wasser, verd. Salzsäure und Wasser gewaschen und bei 120°C im Vakuum getrocknet. Man erhält 260 Teile gelbes Naphtholon mit einem Reingehalt von ca. 98% und einer Ausbeute von 82% d. Th., bezogen auf 5-Aminobenzimidazolon-(2).

## Beispiel 7

In eine Lösung von 110 Teilen 2-Hydroxy-3-naphthoesäure in 250 Teilen wasserfreiem NMP laufen 32 Teile Phosphortrichlorid. Das Reaktionsgemisch erwärmt sich von 25 auf ca. 50°C. Diese Lösung lässt man zu einer Lösung von 75 Teilen wasserfreiem 5-Aminobenzimidazolon-(2) in einem wasserfreien Gemisch von 150 Teilen NMP und 44 Teilen Pyridin zulaufen. Das Reaktionsgemisch erwärmt sich von 25 auf ca. 60°C und wird dann noch weiter auf 140–145°C erhitzt. Dabei kristallisiert das gelbe Naphtholon aus. Man rührt noch 3 Stunden bei dieser Temperatur nach und verdünnt dann mit 120 Teilen iso-Butanol. Das Naphtholon wird filtriert und mit heissem iso-Butanol gewaschen. Man erhält 114 Teile gelbes Naphtholon. Aus dem Destillationsrückstand der Mutterlauge bei der Regeneration von NMP und Pyridin lassen sich noch ca. 40 Teile verunreinigtes Naphtholon isolieren, die dem nächsten Ansatz zugeschlagen werden. Unter diesen Bedingungen erhält man 135–140 Teile reines gelbes Naphtholon = 86% d. Th.

In den folgenden Beispielen wird die Herstellung der gelben Kristallmodifikation des Naphtholons durch Modifikationsumwandlung beschrieben.

Als Ausgangsprodukt dient ein Naphtholon, welches durch Lösen in wässrigem Alkali, Klärung über Aktivkohle und Fällung mit Mineralsäure gereinigt wurde und dessen Röntgenbeugungsspektrum dann durch folgendes Röntgenbeugungsspektrum gekennzeichnet ist (Cu-K$_\alpha$-Linie):

2,4(w), 3,1(w), 4,3(w), 4,6(m), 7,25(vs), 8,0(m), 12,2(w), 13,2(s), 13,4(s), 13,9(s), 14,5(w) und 15,9(m).

Hieraus ergeben sich folgende Netzebenenabstände ($10^{-10}$ m):

18,30; 14,20; 10,20; 9,50; 6,11; 5,55; 3,65; 3,38; 3,33; 3,21; 3,08; 2,81.

### Beispiel 8

136 Teile eines wässrigen Filterkuchens von weissem Naphtholon mit einem Feststoffgehalt von 22%, gekennzeichnet durch die oben angegebenen Netzebenenabstände im Röntgenbeugungsspektrum, werden mit 300 Teilen NMP versetzt. Man destilliert unter Rühren so viel Wasser-NMP-Gemisch ab, bis die Innentemperatur der zähflüssigen Suspension auf 120–130°C gestiegen ist. Nach 2 bis 3 Stunden ist die Suspension dünnflüssig geworden. Gleichzeitig ist ihre Farbe von hellgrau nach schwach gelb umgeschlagen. Man tropft nun bei 100°C 100 Teile Wasser zu, saugt bei Raumtemperatur ab und wäscht mit heissem Wasser nach.

Man erhält 60 Teile eines wässrigen Filterkuchens mit einem Feststoffgehalt von 48,5%. Das getrocknete Produkt besitzt dasselbe Röntgenbeugungsspektrum wie das Produkt nach Beispiel 1.

### Beispiel 9

Beispiel 8 wird mit dem Unterschied wiederholt, dass man an Stelle von NMP 300 Teile Dimethylformamid verwendet. Die Umwandlung des weissen Naphtholons in die gelbe Kristallmodifikation erfolgt bei 110–120°C innerhalb von 2 bis 3 Stunden.

Das Röntgenbeugungsspektrum des Produkts entspricht dem in Beispiel 1 angegebenen Spektrum für die gelbe Kristallmodifikation.

### Beispiel 10

Beispiel 8 wird mit dem Unterschied wiederholt, dass man an Stelle von NMP 300 Teile Dimethylsulfoxid verwendet.

Die Umwandlung des weissen Naphtholons in die gelbe Kristallmodifikation erfolgt bei ca. 110–120°C. Das Röntgenbeugungsspektrum des Produkts entspricht dem in Beispiel 1 angegebenen Spektrum.

### Beispiel 11

136 Teile eines wässrigen Filterkuchens von weissem Naphtholon mit einem Feststoffgehalt von 22% werden mit 300 Teilen NMP und 200 Teilen Wasser im Autoklaven 3 Stunden auf 150°C erhitzt. Nach dem Absaugen bei Raumtemperatur erhält man 62 T. feuchten Filterkuchen (entspr. 29,5 T. getrocknet). Das Produkt stellt laut Röntgenbeugungsspektrum die gelbe Kristallmodifikation des Naphtholons dar.

### Beispiel 12

10 Teile einer getrockneten Probe von weissem Naphtholon werden im offenen Kolben ohne Zusatz eines Lösemittels 3 Stunden auf 300°C erhitzt.

Das Röntgenbeugungsspektrum einer so behandelten Probe ist durch die Reflexe gekennzeichnet, die für die gelbe Kristallmodifikation charakterstisch sind.

Verwendet man an Stelle einer getrockneten Probe von weissem Naphtholon einen wasserfeuchten Filterkuchen, so wird das gleiche Ergebnis erhalten.

### Patentansprüche:

1. 5-(2'-Hydroxy-3'-naphthoyl-amino)-benzimidazolon-(2), gekennzeichnet durch ein Kristallgitter mit folgenden Netzebenenabständen (in $10^{-10}$ m):

3,12; 3,26; 3,37; 3,46; 3,58; 3,75; 4,64; 5,26; 5,43; 5,93; 6,47; 8,74 und 13,10.

2. Verfahren zur Herstellung der Verbindung nach Anspruch 1, dadurch gekennzeichnet, dass man 2-Hydroxy-naphthoesäure mit 5-Amino-benzimidazolon-(2) in einem organischen, polar aprotischen Lösemittel bei Temperaturen über 100°C kondensiert.

3. Verfahren zur Herstellung der Verbindung nach Anspruch 1, dadurch gekennzeichnet, dass man ein durch Kondensation von 2-Hydroxy-3-naphthoesäure und 5-Amino-benzimidazolon-(2) in unpolaren Lösemitteln und Umfällen aus wässrig-alkalischer Lösung mit Säuren erhaltenes Produkt auf Temperaturen von 100 bis 300°C erhitzt.

4. Verfahren nach Anspruch 3, dadurch gekennzeichnet, dass das trockene Produkt erhitzt wird.

5. Verfahren nach Anspruch 3, dadurch gekennzeichnet, dass das Produkt in einem polaraprotischen Lösemittel erhitzt wird.

6. Verfahren nach Anspruch 5, dadurch gekennzeichnet, dass das polar-aprotische Lösemittel N-Methyl-pyrrolidon ist.

7. Verfahren nach Anspruch 6, dadurch gekennzeichnet, dass das Produkt auf Temperaturen bis 200°C erhitzt wird.

8. Verfahren der Verbindung nach Anspruch 1 als Kupplungskomponente für Azopigmente.

**Revendications**

1. 5-(2-hydroxy-3'-naphtoyl-amino)-benzimidazolone-(2), caractérisée en ce que son réseau cristallin présente les distances suivantes (en $10^{-10}$ m) entre plans réticulaires:
3,12; 3,26; 3,37; 3,46; 3,58; 3,75; 4,64; 5,26; 5,43; 5,93; 6,47; 8,74; et 13,10.

2. Procédé pour produire le composé selon la revendication 1, caractérisé en ce qu'on condense l'acide 2-hydroxy-naphtoïque avec la 5-amino-benzimidazolone-(2) dans un solvant organique polaire aprotique à des températures supérieures à 100°C.

3. Procédé pour produire le composé selon la revendication 1, caractérisé en ce qu'on soumet un produit, obtenu par condensation de l'acide 2-hydroxy-3-naphtoïque et de la 5-amino-benzimidazolone-(2) dans des solvantes non-polaires et précipitation, à partir d'une solution alcaline aqueuse, avec un acide, à un chauffage à des températures de 100° à 300°C.

4. Procédé selon la revendication 3, caractérisé en ce qu'on chauffe le produit sec.

5. Procédé selon la revendication 3, caractérisé en ce qu'on chauffe le produit dans un solvant aprotique polaire.

6. Procédé selon la revendication 5, caractérisé en ce que le solvant aprotique polaire est la N-méthyl-pyrrolidone.

7. Procédé selon la revendication 6, caractérisé en ce qu'on chauffe le produit jusqu'à 200°C.

8. Application du composé selon la revendication 1 comme copulant pour la fabrication de pigments azoïques.

**Patent Claims:**

1. 5-(2'-Hydroxy-3'-naphthoylamino)-benzimidazolone-(2), characterized by a crystal lattice having the following interplanar spacings (in $10^{-10}$ m):
3.12; 3.26; 3.37; 3.46; 3.58; 3.75; 4.64; 5.26; 5.43; 5.93; 6.47; 8.74 and 13.10.

2. Process for the preparation of the compound according to claim 1, characterized by condensing 2-hydroxy-naphthoic acid with 5-aminobenzimidazolone-(2) in an organic polar aprotic solvent at temperatures above 100°C.

3. A process for the preparation of the compound according to claim 1, characterized by heating a product obtained by condensation of 2-hydroxy-3-naphthoic acid and 5-aminobenzimidazolone-(2) in unpolar solvents and precipitation from an aqueous alkaline solution with acids to a temperature of from 100 to 300°C.

4. A process according to claim 3, characterized by heating the dry product.

5. A process according to claim 3, characterized by heating the product in a polar aprotic organic solvent.

6. A process according to claim 5, characterized in that the polar aproctic solvent is N-methyl pyrrolidone.

7. A process according to claim 6, characterized by heating the product to temperatures of up to 200°C.

8. Use of the compound according to claim 1 as a coupling component for azo pigments.